# EUROPEAN PATENT SPECIFICATION

(11) **EP 3 798 322 B1**
(45) Date of publication and mention of the grant of the patent: **21.12.2022**
(21) Application number: 19199825.1
(22) Date of filing: 26.09.2019
(51) Int. Cl.: C12R 1/225, A23C 9/12, A23L 33/135

(54) **LACTOBACILLUS REUTERI**
LACTOBACILLUS REUTERI
LACTOBACILLUS REUTERI

(43) Date of publication of application: 31.03.2021
(73) Proprietor: PrecisionBiotics Group Limited, Cork T12 N84F (IE)
(72) Inventor: WALTER, Jens, Cork, T12 A2T3 (IE)
(74) Representative: Weldon O'Brien Ltd.

(56) References cited:
- JANUANA S TEIXEIRA ET AL: "Levansucrase and sucrose phoshorylase contribute to raffinose, stachyose, and verbascose metabolism by lactobacilli", FOOD MICROBIOLOGY, ACADEMIC PRESS LTD, LONDON, GB, vol. 31, no. 2, 5 March 2012 (2012-03-05), pages 278-284, XP028488311, ISSN: 0740-0020, DOI: 10.1016/J.FM.2012.03.003 [retrieved on 2012-03-11]
- TEIXEIRA JANUANA S ET AL: "Functional characterization of sucrose phosphorylase andscrR, a regulator of sucrose metabolism inLactobacillus reuteri", FOOD MICROBIOLOGY, ACADEMIC PRESS LTD, LONDON, GB, vol. 36, no. 2, 1 August 2013 (2013-08-01) , pages 432-439, XP028708760, ISSN: 0740-0020, DOI: 10.1016/J.FM.2013.07.011

## Description

### Field of the Invention

The invention relates to *Lactobacillus reuteri* from a novel phylogenetic clade with unique immune-stimulatory properties and enhanced ecological performance in the human gut.

### Background of the Invention

The gut microbiota is a critical determinant of human health by directly contributing to pathologies, influencing host predisposition to disease. There is now substantial evidence that modern lifestyle and industrialization has resulted in a decrease in the bacterial diversity of the gut microbiota, likely due to a combination of factors such as use of antibiotics, modern clinical practices, sanitation, and change in dietary habits (Blaser & Falkow 2009; Segata, 2015). Increasing incidences of NCDs (non-communicable disease) in all parts of the world are linked to affluent lifestyles and are thought to result, at least in part, from loss of microbial diversity (Haahtela et al., 2015; Hanski et al., 2012; Rook, 2013). The mechanisms for these connections are unknown, and most focus has been on pathogenic microbes in concepts such as the 'hygiene hypothesis' (Jarchum et al., 2011). An alternative, and perhaps more plausible, hypothesis has been proposed that states that during human evolution, microbes have evolved into an essential role in regulating our immune system. The microbes involved are not infections, but friendly, symbiotic microbes which make up our human microbiome. These are acquired by exposure to other humans or animals and microbiota from our natural environment. Researchers now agree that a range of lifestyle changes including an increase in Caesarean section (C-section) births, less breast-feeding, smaller family sizes, and less time outdoors are underlying causes of reduced exposure to friendly microbes, whilst altered diet and antibiotics have adverse effects on the composition of the microbiome.

There is therefore a need to improve the bacterial diversity of the gut microbiota.

*L. reuteri* has been shown to be one of the truly indigenous bacteria of the human GI tract (Sinkiewicz, 2010). It naturally colonizes a wide range of vertebrates, including humans, pigs, hamsters, mice, rats, dogs, sheep, cattle, and different species of birds (including chickens), and it has also been found in the human urogenital tract and breast milk. (Mitsuoka, 1992).

The prevalence of *L. reuteri* is now very low in western human populations, where the species is only occasionally found (Walter J, 2008) There is some evidence that the prevalence of *L*. *reuteri* in human fecal samples was higher in the middle of the past century. Gerhard Reuter and Tomonari Mitsuoka, who in the 1960s and 1970s intensively studied the *Lactobacillus* biota of the human digestive tract, reported that *L. reuteri* was then one of the dominant lactobacilli and regularly detected (Mitsuoka T., 1992; Reuter G., 2001). The low prevalence in humans in more recent studies suggests a reduction of the *L. reuteri* population size during the past 50 years.

The term a "clade" (from Ancient Greek: κλάδος, klados, "branch"), also known as monophyletic group, is defined as a group of organisms that consists of a common ancestor and all its lineal descendants, and represents a single "branch" on the "tree of life".

The common ancestor may be an individual, a population, a species (extinct or extant), and so on right up to a kingdom and further. Clades are nested, one in another, as each branch in turn splits into smaller branches. These splits reflect evolutionary history as populations diverged and evolved independently.

The species *L. reuteri* represents the *L. reuteri* group in the heterofermentative clade of lactobacilli (Duar et. A1. 2017b). *L. reuteri* was thought to be well characterzed genetically and physiologically, and metabolic traits that contribute to its ecological fitness in cereal and intestinal ecosystems are well understood (Zhao and Ganzle, 2018).

Human-derived *L. reuteri* strains belong to two distinct MLSA clades, designated clade II and VI. Clade II contains most human intestinal isolates and clusters together with isolates from ruminants, while human strains in clade VI are closely related to isolates from chickens (Oh, et al. 2010).

### Summary of the Invention

The invention provides a *Lactobacillus reuteri* strain having a sequence identity of 97.5% or more with the DNA sequence of *Lactobacillus reuteri* strain having NCIMB accession number 42835.

Strain 42835 is a *Lactobacillus reuteri* from a novel phylogenetic clade with unique immune-stimulatory properties and enhanced ecological performance in the human gut.

A person skilled in the art would appreciate that strains can be identified by DNA sequence homology analysis with strain NCIMB 42835. Strains having a close sequence identity with strain NCIMB 42835 without demonstrable phenotypic or measurable functional differences are within the scope of the invention. A strain with a sequence identity (homology) of 97% or more, 97.5% or more, 97.75% or more, 98% or more, 98.25% or more, 98.5% or more, 98.75% or more, 99% or more, 99.25% or more, 99.5% or more, 99.75% or more with the DNA sequence of NCIMB 42835 are within the scope of the invention. Sequence homology may be determined using on-line homology algorithm "BLAST" program, publicly available at http://www.ncbi.nlm.nih,gov/BLAST/.

In one embodiment the strain is a human symbiont.

The invention provides *Lactobacillus reuteri* strain having NCIMB accession number 42835 or mutants or variants thereof.

The mutant may be a genetically modified mutant.

The variant may be a naturally occurring variant.

The strain may be in the form of a biologically pure culture.

Also provided is an isolated strain of *Lactobacillus reuteri* (NCIMB 42835).

The strain may be a probiotic.

In one case the strain is in the form of viable cells.

In another case the strain is in the form of non-viable cells.

The *Lactobacillus reuteri* strain NCIMB 42835 may be present in the formulation in an amount of more than 10⁶ cfu, typically from 10⁷ to 10¹⁰, typically from 10⁸ to 10⁹ cfu per dose. In one case the *Lactobacillus reuteri* strain NCIMB 42835 is present in the formulation in an amount of about 1×10⁹ cfu per dose.

Bacterial viability reflects the number of culturable bacteria within a sample, i.e. the number of bacteria which retain the ability to reproduce when grown under optimal conditions (Viable cells). Put another way, viability reflects the number of individual bacterial cells which retain the ability to replicate into larger bacterial colonies (colony forming units (CFUs)).

Viability is commonly determined using plate-counting methods, whereby a bacterial sample is diluted and then incubated on an agar plate containing the necessary nutrients for growth. Viability is then calculated from the number of bacterial colonies identified on a plate. Such methods are summarized in Modern Food Biology 2005 7th edition, James Monroe Jay, Martin J. Loessner, David A. Golden, Springer Science, New York.

Whilst plate-counting gives a good indication of viability, it does not encompass all living bacterial cells in the sample. (Kell, Douglas B., et al. "Viability and activity in readily culturable bacteria: a review and discussion of the practical issues. "Antonie van Leeuwenhoek73.2 (1998): 169-187).

Samples will also contain "viable but non-culturable" (VBNC) cells which remain metabolically active but have lost the ability to replicate at the time of analysis by plate count, and thus despite being alive will not form CFUs. Finally, samples will also contain dead cells. These two groups can be grouped together as "Non-Viable cells". Therefore, Non-viable cells are the inverse of Viable cells i.e. all those cells which have lost the ability to replicate when tested.

All samples containing Viable cells will also contain Non-Viable cells, therefore the definition of a Viable cell culture is clarified using CFU measurements.

All Non-Viable samples will contain at least VNBCs and possibly small amounts of Viable cells. Industry standard lower level detection limits of 10³ CFU/g viable cells allow for the inherent process variability caused by the presence of a certain number of VBNCs/Viable cells in Non-Viable samples.

In some embodiments, such as, but not limited to, special sterile food products or medicaments a non-replicating form of a probiotic strain may be preferable. For example, at least 95%, preferably at least 97%, more preferably at least 99% of the *Lactobacillus reuteri* strain can be non-replicating in the composition.

In one case the strain is significantly immunomodulatory following oral consumption in humans.

In one embodiment the strain is in the form of a bacterial broth.

In another embodiment the strain is in the form of a freeze-dried powder.

The invention provides a formulation which comprises a strain of the invention.

The formulation may further comprise another probiotic material.

The formulation may further comprise a prebiotic material.

The formulation may further comprise an ingestable carrier.

In one case the ingestable carrier is a pharmaceutically acceptable carrier such as a capsule, tablet or powder.

In another case the ingestable carrier is a food product such as acidified milk, yoghurt, frozen yoghurt, milk powder, milk concentrate, ice cream, cheese spreads, dressings or beverages.

In some embodiments the formulation further comprises a protein and/or peptide, in particular proteins and/or peptides that are rich in glutamine/glutamate, a lipid, a carbohydrate, a vitamin, mineral and/or trace element.

In one case the strain is present in an amount of more than 10⁶ cfu per gram of the formulation.

The formulation may further comprise an adjuvant.

The formulation may further comprise a bacterial component.

The formulation may further comprise a drug entity.

The formulation may further comprise a biological compound.

The invention also provides a foodstuff comprising a strain or a formulation of the invention.

Also provided is a medicament comprising a strain or a formulation of the invention.

In one embodiment the formulation further comprises a carbohydrate.

The formulation may comprise an oligosaccharide which may be non-digestible.

In one case the oligosaccharide comprises raffinose.

Also described is a method for enhancing the microbiota of an individual who is a member of industrialised human society comprising the step of administering a strain of *Lactobacillus reuteri* which is isolated from an individual who is not a member of industrialised human society.

The microbiota may be the gut microbiota.

The strain may be a strain as defined herein.

The strain may be a component of a formulation as defined herein.

The method may comprise administering a carbohydrate substrate for the strain. The substrate may comprise an oligosaccharide which may be non-digestible. In one case the oligosaccharide comprises raffinose.

The substrate may be administered at the same time or separately from the strain.

It will be appreciated that the strains of the invention may be administered to animals (including humans) in an orally ingestible form in a conventional preparation such as capsules, microcapsules, tablets, granules, powder, troches, pills, suppositories, suspensions and syrups. Suitable formulations may be prepared by methods commonly employed using conventional organic and inorganic additives. The amount of active ingredient in the medical composition may be at a level that will exercise the desired therapeutic effect.

The formulation may also include a bacterial component, a drug entity or a biological compound.

In addition a vaccine comprising the strains of the invention may be prepared using any suitable known method and may include a pharmaceutically acceptable carrier or adjuvant.

The invention also includes mutants and variants of the deposited strains. Throughout the specification the terms mutant, variant and genetically modified mutant include a strain whose genetic and/or phenotypic properties are altered compared to the parent strain. Naturally occurring variant includes the spontaneous alterations of targeted properties selectively isolated. Deliberate alteration of parent strain properties is accomplished by conventional *(in vitro)* genetic manipulation technologies, such as gene disruption, conjugative transfer, etc. Genetic modification includes introduction of exogenous and/or endogenous DNA sequences into the genome of a strain, for example by insertion into the genome of the bacterial strain by vectors, including plasmid DNA, or bacteriophages.

Natural or induced mutations include at least single base alterations such as deletion, insertion, transversion or other DNA modifications which may result in alteration of the amino acid sequence encoded by the DNA sequence.

The terms mutant, variant and genetically modified mutant also include a strain that has undergone genetic alterations that accumulate in a genome at a rate which is consistent in nature for all micro-organisms and/or genetic alterations which occur through spontaneous mutation and/or acquisition of genes and/or loss of genes which is not achieved by deliberate *(in vitro)* manipulation of the genome but is achieved through the natural selection of variants and/or mutants that provide a selective advantage to support the survival of the bacterium when exposed to environmental pressures such as antibiotics. A mutant can be created by the deliberate *(in vitro)* insertion of specific genes into the genome which do not fundamentally alter the biochemical functionality of the organism but whose products can be used for identification or selection of the bacterium, for example antibiotic resistance.

### Brief Description of the drawings

The invention will be more clearly understood from the following description thereof given by way of example only with reference to the accompanying drawings in which;-
Fig. 1 is an illustration of a phylogenetic tree of new and published *Lactobacillus reuteri* genomes (n = 54) derived from multiple hosts. The phylogenetic tree was constructed based on the alignments of all core genes (n = 639). Genealogy inferred by ClonalFrame from MLSA data (Bayesian method with exclusion of recombinant regions);
Fig. 2 illustrates the differences in gut microbiota in rural Papua New Guineans and individuals living in the United States;
Fig. 3 illustrates the presence of 47 species-level Operational Taxonomic Units (OTUs) in the gut of rural Papua New Guineans that are absent from the gut microbiota on North Americans of which *L. reuteri* is one. The figure highlights the presence of high levels of *L. reuteri* in the gut microbiota of rural Papua New Guineans whereas *L. reuteri* is absence from the gut microbiota of individuals living in the United States;
Fig. 4 illustrates the induction profile of TNF-α in PBMC after in vitro stimulation with one concentration of *L. reuteri* DSM 20016, *L. reuteri* ATCC PTA 6475, *L. reuteri* DSM 17938, *L. reuteri* Strain 2 and *L. reuteri* NCIMB 42835;
Fig. 5 illustrates the induction profile of TNF-α in PBMC after in vitro stimulation with increasing concentrations of *L. reuteri* DSM 20016, *L. reuteri* ATCC PTA 6475, *L. reuteri* DSM 17938, *L. reuteri* Strain 2 and *L. reuteri* NCIMB 42835;
Fig. 6 illustrates the induction profile of IL-10 in PBMC after in vitro stimulation with one concentration of *L. reuteri* DSM 20016, *L. reuteri* ATCC PTA 6475, *L. reuteri* DSM 17938, *L. reuteri* Strain 2 and *L. reuteri* NCIMB 42835;
Fig. 7 illustrates the induction profile of IL-6 in PBMC after in vitro stimulation with one concentrations of *L. reuteri* DSM 20016, *L. reuteri* ATCC PTA 6475, *L. reuteri* DSM 17938, *L. reuteri* Strain 2 and *L. reuteri* NCIMB 42835;

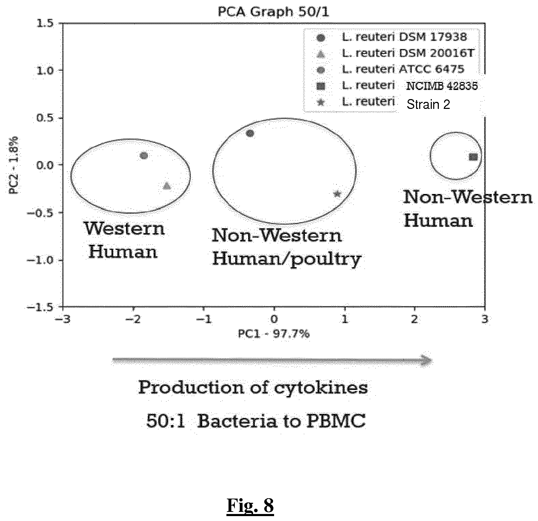

Fig. 8 is a Principle component analysis (PCA) plot of cytokine response of one dose of *Lactobacillus reuteri* strains in the PBMC assay;

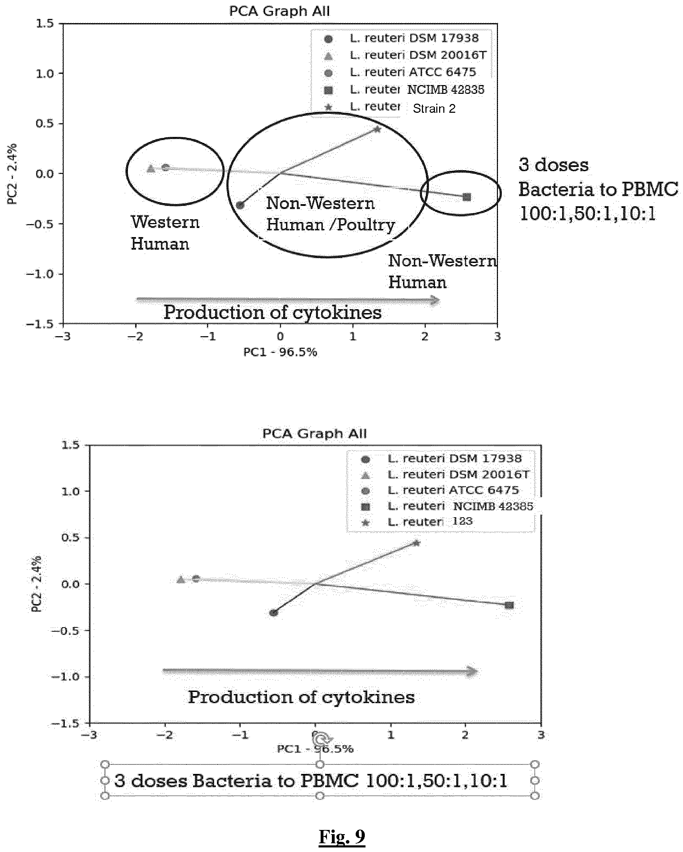

Fig. 9 is a Principle component analysis (PCA) plot of cytokine response of three doses of *Lactobacillus reuteri* strains in the PBMC assay;
Fig. 10 illustrates growth curves of *L. reuteri* NCIMB 42835 using raffinose or glucose as the sole carbohydrate in basal MRS growth media (3% (m/v)) and demonstrates the preference of *L. reuteri* NCIMB 42835 for raffinose over glucose as a growth substrate in B-MRS. Statistical analysis of OD600 values were performed by GraphPad Prism 5.0 with two-way ANOVA, and P< 0.05 was considered as significant. ^{∗∗∗}: P<0.001; ^{∗∗}: P<0.01; ^{∗}: P<0.05;
Fig.11 illustrates the cell numbers of *Lactobacillus reuteri* in fecal samples determined by quantitative plating after a one-time administration of around 10¹⁰ cells of *L. reuteri* at day 4. Days refer to time after the diet switch to a non-western (designed) diet. Data are presented as mean ± SE of log10 of CFU. Within the same day, cell numbers of each treatment labelled with different letters are significantly different based on repeated-measures two-way ANOVA with p < 0.10; and
Fig.12 illustrates the cell numbers of *Lactobacillus reuteri* in fecal samples determined by quantitative plating. Cell numbers of *L. reuteri* NCIMB 42835 (A) and *L. reuteri* DSM 20016 (B). Data are presented as mean ± SE of log10 of CFU. Within the same day, cell numbers between designed non-western diet and usual diet labelled with ^{∗} are significantly different based on paired t-test with p < 0.10.

### Detailed Description of the Invention

A deposit of *Lactobacillus reuteri* strain 1 strain was made at the National Collections of Industrial and Marine Bacteria Limited (NCIMB) Ferguson Building, Craibstone Estate, Bucksburn, Aberdeen, AB21 9YA, Scotland, UK on September 29, 2017 and accorded the accession number NCIMB 42835.

### Examples

The following examples further describe and demonstrate embodiments within the scope of the invention.

### A new phylogenetic Clade

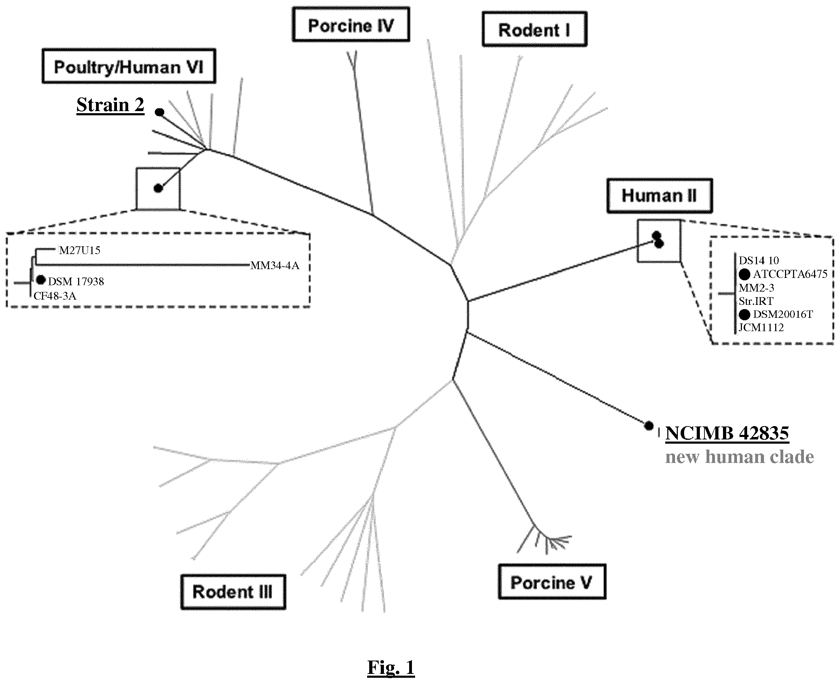

*L. reuteri* was regularly detected in humans in studies conducted around 1960 but is very rarely found in contemporary humans, suggesting a recent decline of *the L. reuteri* population in Westerners (Walter et al., 2011). *L. reuteri* is a real symbiont of vertebrates that has evolved a high level of specificity with its respective host species, pointing to a tight relationship that was maintained over evolutionary times (Oh et. al., 2010; Frese et al., 2011; Duar et al., 2017; Frese et al., 2013). Importantly, *L. reuteri* exerts substantial benefits towards host immune functions and development, as demonstrated in a number of high impact publications (Zelante et al. 2013; Buffington et al. 2016; Lamas et al. 2016; He et al. 2017). However, there are less than 20 genomes of western *L. reuteri* strains available in international databases. Most human isolates cluster in one phylogenetic lineage (linage II) (Oh et. al., 2010). The population structure of this lineage is characterized by a very low level of diversity, showing <50 Single Nucleotide Polymorphisms (SNPs) over the available genomes compared to other species (Fig. 1). Overall, these findings suggest a recent decline of the *L. reuteri* population in Westerners.

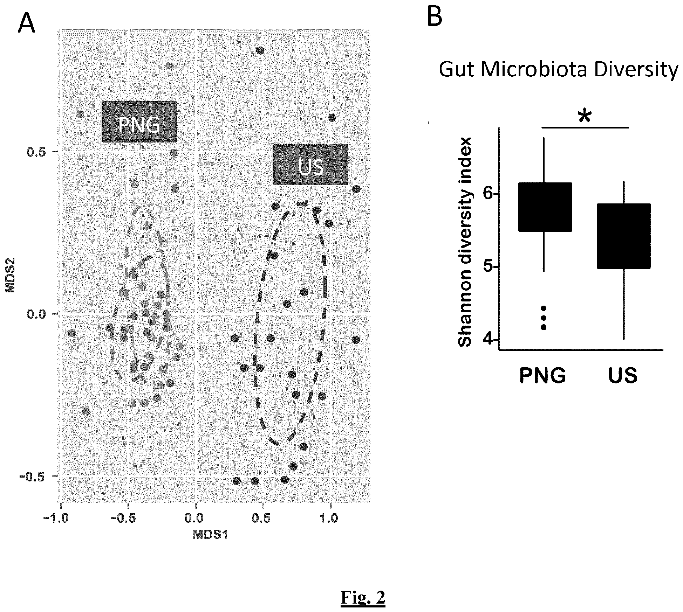

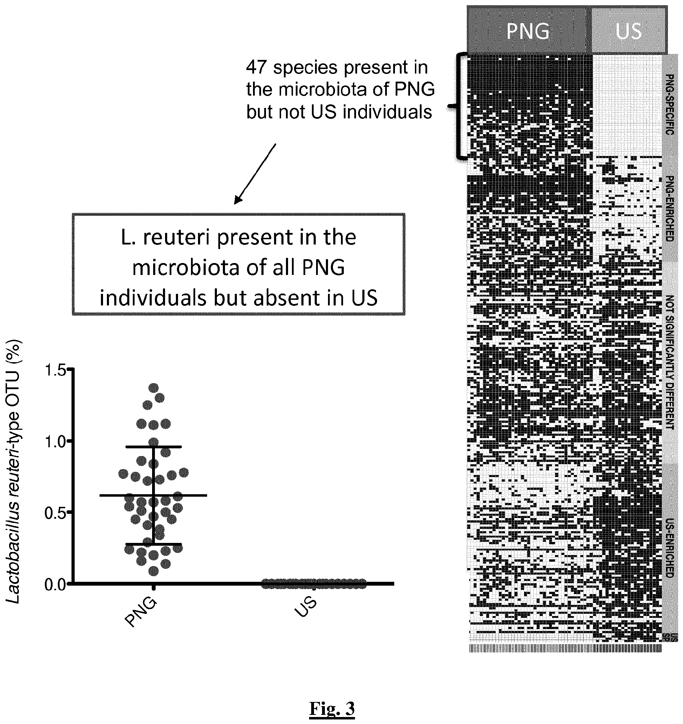

Fig. 2 illustrates the gut microbiota in rural Papua New Guineans. There is now substantial evidence that modern lifestyle and industrialization has resulted in substantial decrease in the bacterial diversity of the gut microbiota, likely due to a combination of factors such as use of antibiotics, modern clinical practices, sanitation, and change in dietary habits (Blaser & Falkow 2009; Segata, 2015). Fig. 2a shows that the gut microbiota is significantly different between Papua New Guineans and North Americans. Fig. 2b shows the higher diversity in the fecal microbiota of individuals from rural tribes in Papua New Guinea. Fig. 3 illustrates that 47 species-like OTUs are completely undetectable in the gut microbiota of North Americans. This work confirms the overall premise of 'microbiome depletion' in western society. Interestingly, one species detectable in every Papua New Guinean individual by 16S rRNA sequencing but not in a single US control is *Lactobacillus reuteri* (Fig. 3). Western non-communicable diseases, such as Autism and IBD, are virtually absent in Papua New Guinea. We have investigated isolates of *L. reuteri* that were obtained from fecal samples of rural Papua New Guineans and identified a new *L. reuteri* strain that differs significantly from *L. reuteri* strains isolated from western humans. *L. reuteri strains* from tribespeople in Papua New Guinea have never been isolated or characterized before.

Genomic analysis and comparison of the *L. reuteri* strains isolated from rural Papua New Guineans to *L. reuteri* strains from western humans indicates that the genome sequence of *L. reuteri* NCIMB 42835 is >2.5% different from any known human *L. reuteri* based on Average Nucleotide Identity (ANI) (Table 1). In particular, *L. reuteri* NCIMB 42835 does not fall within any of the established phylogenic linkages within the *L. reuteri* phylogenic tree (Fig 1). Thus *L. reuteri* NCIMB 42835 represents a newly identified clade.

In contrast, *L. reuteri* strain 2 which was also isolated from rural Papua New Guineans belongs to clade VI.

**Table 1**

| Western human Strains | Non-western *L. reuteri* NCIMB 42835 Average Nucleotide Identity |
|---|---|
| *L. reuteri* MM2-3 | 96.71 |
| *L. reuteri* MM4-1A | 97.42 |
| *L. reuteri* DSM20016 | 96.71 |
| *L. reuteri* str_IRT | 96.72 |

Average nucleotide identity (ANI; below diagonal) and 16S rRNA gene similarity (above diagonal) among *L. reuteri* strains - Table 2.

**Table 2**

| ANI1 | DSM 20016T | NCIMB 42835 | ATCC PTA 6475 | DSM 17938 |
|---|---|---|---|---|
| DSM 20016T | -- | 99.81 | 99.87 | 99.50 |
| NCIMB 42835 | 97.74 | -- | 99.94 | 99.68 |
| ATCC PTA 6475 | 100.00 | 97.42 | -- | 99.62 |
| DSM 17938 | 96.87 | 96.50 | 96.87 | -- |

| | | | | |
|---|---|---|---|---|
| ***Note:*** ¹ANI was calculated based on sequences of concatenated core genes (n = 639) from novel and published *L. reuteri* genomes (n = 54). ²The complete genome sequence of SD2112 (without plasmid sequences) was used to represent the genomic characteristics of DSM 17938. | | | | |

**Method:** The Roary pipeline was applied to identify core genes based on annotated assemblies of novel and published *L. reuteri* genomes (n = 54). Average nucleotide sequence identity (ANI) values among 7 different strains were calculated based on these concatenated core genes in JSpeciesWS (http://ispecies.ribohost.com/jspeciesws/#analyse/) with BLAST algorithm. To obtain full-length 16S rRNA gene sequences, the assembly of each strain was aligned to the 16S rRNA gene sequence of DSM 20016^{T} using BLAST, and those aligned sequence fragments were further assembled manually. Similarity values among 16S rRNA genes were calculated using BLAST.

### NCIMB 42835 induces different cytokine responses in human immune cells

We have complemented these genomic comparisons with functional data on the effects of *L. reuteri* strains on cytokine production by human immune cells (PBMCs). The analysis shows that strains from the same phylogenetic lineage show similar responses, indicating that the evolutionary history of *L. reuteri* strains influences their interactions with the host in agreement with previous findings (Spinler et al., 2014). The findings further demonstrate clear differences between NCIMB 42835 and strains from the other two sub-populations of human-derived probiotic *L. reuteri.*

Studies of neonates born in areas of the developing world with very high microbial burden, such as Papua New Guinea (PNG), show extensive differences in neonatal immune function compared with newborns in highly developed regions, such as Australia (Lisciandro et al., 2012a; 2012b). At the end of gestation, antigen-presenting cells from PNG neonates already show much higher baseline expression of markers of activation (HLA-DR and CD86) and inhibition (immunoglobulin-like transcripts 3 and 4) compared with Australian newborns. However, on activation, antigen- presenting cells from PNG cord blood are relatively quiescent with reduced activation and antigen processing and evoke an attenuated T-cell response compared with Australian neonates. Although there are many environmental differences between these settings, microbial burden is one of the most striking, and the more tolerogenic responses of PNG newborns might protect against the development of harmful inflammatory responses in early life. In this respect, it is important to emphasize that *L. reuteri* strains have been shown to be immune-modulatory and induce regulatory T cells in rodent studies (Liu et al., 2013; 2017).

These and other experiments of nature all strongly support the role of the prenatal maternal environment in early immune programming and raise the challenging question of how to harness this to improve immune health in the setting of globally increasing propensity for allergy and other chronic inflammatory diseases.

Studies examining different strains have shown that clinical effects are strain-specific (Wickens et al., 2008). This means that interventions such as supplementation with the novel NCIMB 42835 *L reuteri* from Papua New Guinea natives into a depleted Western Microbiome may have health associated Immune responses (Abrahamson et al., 2012; Van Nimwegen et al 2011; Bisgard et al., 2011).

*Lactobacillus reuteri* DSM 20016 is the type strain for *Lactobacillus reuteri,* and *Lactobacillus reuteri ATCC* PTA 6475 is a commercially available strain and both were isolated from western subjects and are from the Human II clade (Fig 1). *Lactobacillus reuteri* DSM 17938 is a commercially available strain which was isolated from the breast milk of a Peruvian Indian and this strain falls into poultry/human clade VI. We isolated a novel strain from native Papua New Guinean subjects, *Lactobacillus reuteri* NCIMB 42835, who live a non-industrialized lifestyle. The aim of this study was to determine if these strains had a different cytokine profile when incubated with PBMCs from healthy volunteers *in vitro.*

### PBMC isolation

Peripheral venous whole blood from 3 healthy donors (males between 30 and 45 years old) was collected in sterile EDTA tubes, mixed few times by inversion and diluted 1/1 with sterile PBS. 20 mL of Ficoll into a new sterile 50 mL conical tube was aliquoted for each donor. For each donor, 25 ml of the diluted EDTA-whole blood was layered on top of the Ficoll aliquoted in step 2. Tubes were centrifuged at 800 g for 30 minutes at 4°C with brake off. The white buffy coat (PBMC layer) below the plasma was collected and cells were washed twice with sterile PBS. The PBMC were resuspended in fresh warm RPMI 10%FBS + 1% Penicillin/streptomycin and counted using an automatic cell counter. The number of cells for each donor was adjusted at 1^{∗}10⁶ per ml and aliquoted in 24 well-plates.

### Strain preparation

Frozen and lyophilized *L. reuteri* strains were briefly centrifuged and resuspended with 10 ml of warm RPMI under sterile conditions to make the stock concentration. The top, middle and low concentrations were prepared (100:1) top dose: 1ml of stock concentration was added to 4.5ml of complete RPMI. (50:1) medium dose: the top dose was diluted 1:1 with complete RPMI. To make the (10:1) lower dose: Top dose was diluted 1:9.

### Incubation and controls

A PBMC suspension of 5×10⁵ cells in 500µl was aliquoted in 24 wells. PBMC's were incubated with 50µl of strain for 24 hours, in humidified incubator at 5% CO₂ (37 °C, 95% air, 100% humidity). Supernatants were collected and centrifuged to pellet cells and the clean cell-free conditioned media were moved in a new Eppendorf and stored immediately at -80C.

The controls are represented as: PBMC + Vehicle (strain cryoprotectant).

### Luminex multiplex immunoassays

Samples were diluted 1/100 in Assay diluent immediately before the assay. For each well, 50µL of standard or diluted samples were dispensed in the wells + 50µL.

The analytes to be measured were 3 cytokines IL-10, TNF-α, IL-6). The plate was incubated in accordance with the Manufacturer's protocol and read in a Luminex MAGPIX analyser.

*Lactobacillus reuteri* NCIMB 42835 induced more TNF-α, IL-10, IL-6 from PBMCs than all the other *L. reuteri* (Figure 1, 3, 4). When analysing TNF-α induction in PBMCs after all 3 doses of *L. reuteri* NCIMB 42835 has a unique profile which shows a dose-dependent induction of this cytokine (Figure 2).

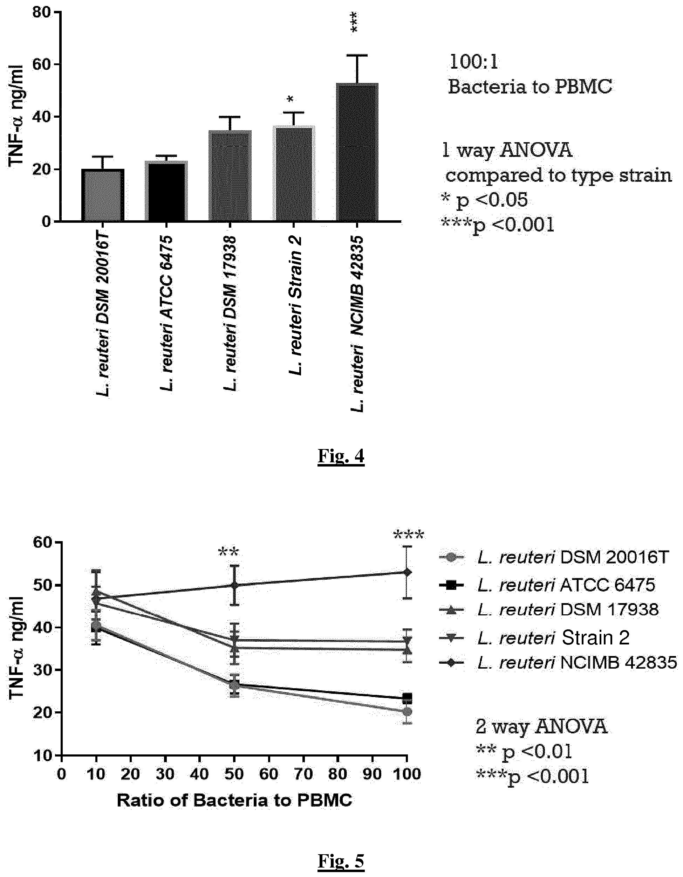

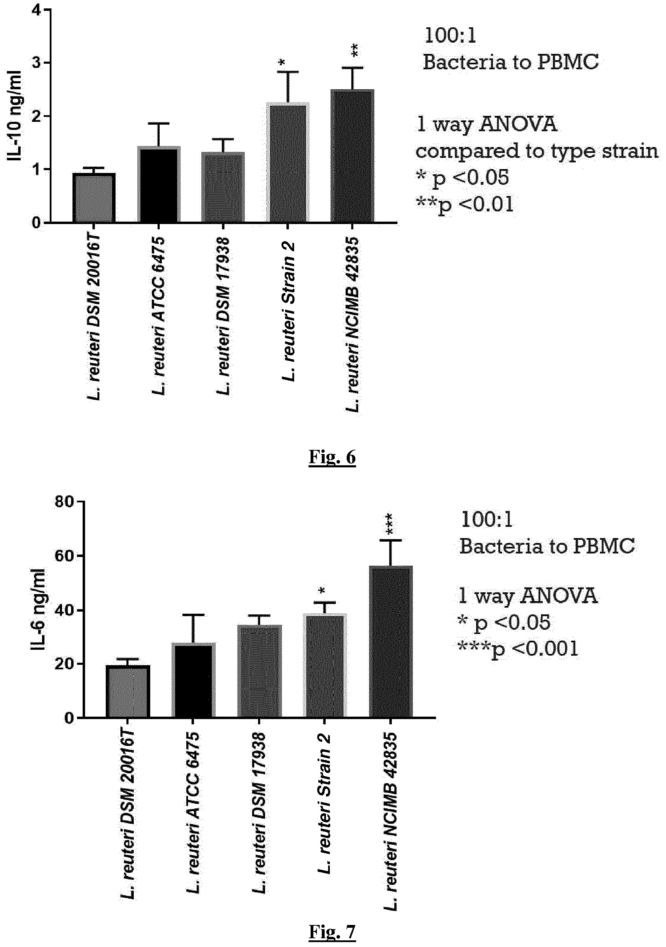

Principle component analysis (PCA) is an analysis of principal components of data to visualize the underlying structure of the data where there is the most variance and separation from each other. PCA on the all cytokine data from one dose or from 3 doses of the *L. reuteri* strains found that NCIMB 42835 is the furthest dot to the right of the plot hence showing the highest induction of cytokines. It is the furthest away from the two western strains and it is clearly separated from the other two non-western strains as well which seem half way between the western strains and NCIMB 42835 Figure 5, 6). The cytokine data agrees with the phylogenetic data showing the NCIMB 42835 is a unique isolate with a strain specific effect on key cytokines in human cells.

### Preferential use of certain carbohydrates by L. reuteri NCIMB 42835

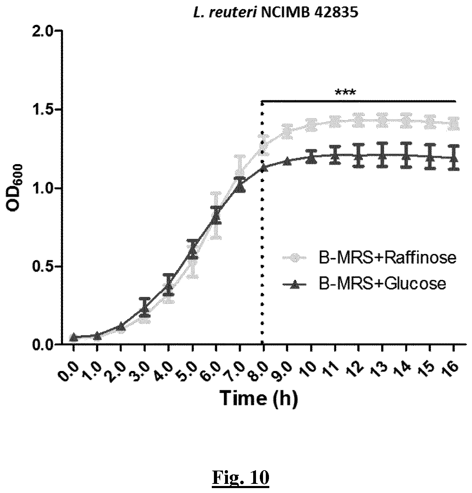

Carbohydrates such as raffinose are present at very low amounts in western diet, while being abundant in the diet of Papua New Guineans, a population that consumes a predominantly plant-based diet. As mentioned previously, *L. reuteri* are highly adapted and loss of important growth substrates and important traits in western isolates may explain the decline of the *L. reuteri* population in Westerners. The use of raffinose to support the growth *L. reuteri* in the gut has not been previously reported. Figure 10 shows higher growth for *L. reuteri* NCIMB 42835 on raffinose compared to glucose.

These results demonstrate that raffinose is an excellent growth substrate for the non-western *L*. *reuteri* strains with superior growth achieved compared to western strain. The use of raffinose in combination with a non-western *L. reuteri* could help to support the re-establishment of this beneficial strain in the gut microbiota of western societies.

### L. reuteri NCIMB 42835 shows higher persistence than L. reuteri DSM20016T (type strain) in the human gut and benefits from a diet high in raffinose.

To determine if *L. reuteri* NCIMB 42835 shows an adaptation to the human gut and to dietary raffinose, the Walter lab is in the process of conducting a human study that assesses the performance of the strain in the human gut. The goal of this study is to demonstrate that a bacterial species dominant in the non-westernized microbiome can be 'reintroduced' in the gut of Canadians fed a diet designed to promote the growth of the bacteria.

**Human trial.** Participants were recruited from the University of Alberta Campus and informed consent was obtained. In a three-arm trial design, the effect of two strains of *L. reuteri* (NCIMB 42835 and DSM 20016T) were compared to a placebo group. The impact of a non-western diet is tested as a cross-over design in all study arms. Healthy men and pre-menopausal, non-pregnant and non-lactating women, 18-45 years of age, with a BMI between 20-29.9 kg/m², and no recent history (<3 months) of antibiotic use (N= 30). Participants were stratified based on sex and randomly assigned to 1 of the 3 groups. Groups 1 and 2 will receive the *L. reuteri* strains provided in water for direct consumption, as described by Duar et al. (Duar et al., 2017). Both strains were provided about at a dose of around 10¹⁰ viable cells, which is a dose by which *L*. *reuteri* is easily tolerated by humans (Duar et al., 2017). Group three will received a placebo (2 g maltodextrin diluted in water), which is known not to impact microbiome. All groups received a single dose of the probiotic or placebo on day 4 and day 39 of the intervention. Participants will be asked to maintain their usual diet during the one-week screening period. After one week, half of the subjects per arm were assigned to follow the 'non-western diet' (or designed diet), while half continued to consume their usual diet for three weeks (usual diet). After a 2-week washout, participants switched diets, for the second 3-week period of the study. Participants attended a total of 12 clinic visits throughout the course of the study. The baseline visit will occur the day before the commencement of each diet period (days 0 and 35); during these visits, anthropometric, body composition measured by bioelectrical impedance analysis (BIA), blood pressure measurements, blood and stool samples were collected, and participants completed questionnaires on perceived stress and GI tolerance. On study days 4 and 39, participants received the *L. reuteri* strain or placebo according to which group they have been assigned. In addition to the scheduled visits, participants were asked to provide stool samples approximately every two days during each diet period.

**Diet.** The Walter Lab, in collaboration with other labs at the University of Alberta, designed a dietary intervention that resembles that of individuals following an agrarian lifestyle, while specifically selecting food products that provide indigestible substrates for *L. reuteri.* While a typical western diet is characterized by high intake of red and processed meats, eggs refined grains and sugars, diets of individuals native to Papua New Guinea are comparatively higher in plant based foods, fibre, and carbohydrates, and lower in fat and animal sources of protein (Martinez et al, 2015). Specifically, raffinose is common in the Papua New Guinean diet, but rare in the western diet, and is predicted to support the growth of *L. reuteri* in the human gut (see our preliminary data). While assigned to the non-western (designed) diet period of the study, participants were provided with fully prepared, pre-packaged breakfasts, lunches, dinner and snacks composed of food products that resemble the amounts and type of carbohydrate sources consumed by individuals from an agrarian society (e.g. yam, sweet potato, and cassava). Additionally, the diet was conservative in the amount of animal protein provided (frequency of once daily or less) emphasizing plant sources of protein such as fava beans and split peas.

**Production of *L. reuteri* strains for consumption.** *L. reuteri* strains were prepared for consumption in Walter lab under food grade conditions, and cell numbers were established prior to the harvest and the dose was standardized as described by Duar et al. (2017) to around 10⁹ cells per ml and provided to the subjects in spring water.

### Determination of L. reuteri cell numbers in fecal samples.

Fecal samples were collected every 2 days (twice before and for two weeks after consumption of the *L. reuteri* or the placebo). Absolute quantification of the *L. reuteri* strains in fecal samples by bacterial culture using LRIM agar. This agar has been shown to be sufficiently selective to quantitatively isolated *L. reuteri* from human fecal samples (Duar et al., 2017).

### Preliminary Findings:

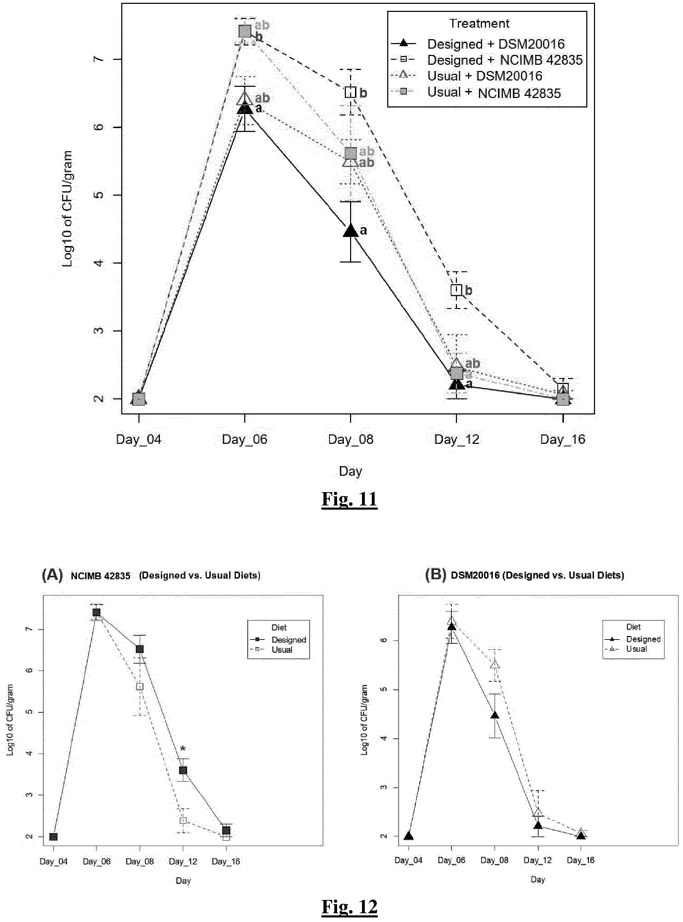

As shown in Fig. 11, strain NCIMB 42835 reaches more than 10 fold the cell number when compared with DSM 20016T two days after administration. Numbers drop afterwards but stay higher NCIMB 42835. In addition, the diet switch to a non-western high plant diet (designed diet) leads to a higher persistence at 8 days after administration (Fig. 12 A+B). These findings show that strain NCIMB 42835 shows adaptations to the human gut and benefits from a diet that resamples that of a non-industrialized agrarian human population.

Given that modern lifestyle is associated with substantial increase in chronic disease, redressing lifestyle-induced microbiome depletion has tremendous therapeutic potential. Isolates of *L. reuteri,* originating from rural Papua New Guinea, when put back in the gut of individuals living in industrialized societies, will likely exert health benefits and could help prevent western non-communicable diseases such as autism. In the invention this important symbiont (a species with a long-term evolutionary relationship which results in a mutual benefit) is isolated from non-industrial individuals and demonstrates unique properties. Introduction into the diet of industrialized or westerns humans, which includes its use as a probiotic with and without the parallel administration of substrates such as raffinose has not been proposed before.

It is expected that there will be particular advantages to enhancing the microbiota of infants, young children, a pregnant female or a mother post childbirth.

It will be appreciated that the strains of the invention may be administered to animals (including humans) in an orally ingestible form in a conventional preparation such as capsules, microcapsules, tablets, granules, powder, troches, pills, suppositories, suspensions and syrups.

Suitable formulations may be prepared by methods commonly employed using conventional organic and inorganic additives. The amount of active ingredient in the medical composition may be at a level that will exercise the desired therapeutic effect.

The formulation may also include a bacterial component, a drug entity or a biological compound.

In addition a vaccine comprising the strains of the invention may be prepared using any suitable known method and may include a pharmaceutically acceptable carrier or adjuvant.

The human immune system plays a significant role in the aetiology and pathology of a vast range of human diseases. Hyper and hypo-immune responsiveness results in, or is a component of, the majority of disease states. One family of biological entities, termed cytokines, are particularly important to the control of immune processes. Perturbances of these delicate cytokine networks are being increasingly associated with many diseases. These diseases include but are not limited to inflammatory disorders, immunodeficiency, inflammatory bowel disease, irritable bowel syndrome, cancer (particularly those of the gastrointestinal and immune systems), diarrhoeal disease, antibiotic associated diarrhoea, paediatric diarrhoea, appendicitis, autoimmune disorders, Alzheimer's disease, rheumatoid arthritis, coeliac disease, diabetes mellitus, organ transplantation, bacterial infections, viral infections, fungal infections, periodontal disease, urogenital disease, sexually transmitted disease, HIV infection, HIV replication, HIV associated diarrhoea, surgical associated trauma, surgical-induced metastatic disease, sepsis, weight loss, anorexia, fever control, cachexia, wound healing, ulcers, gut barrier function, allergy, asthma, respiratory disorders, circulatory disorders, coronary heart disease, anaemia, disorders of the blood coagulation system, renal disease, disorders of the central nervous system, hepatic disease, ischaemia, nutritional disorders, osteoporosis, endocrine disorders, epidermal disorders, psoriasis and acne vulgaris. The effects on cytokine production are specific for the probiotic strain examined. Thus specific probiotic strains may be selected for normalising an exclusive cytokine imbalance particular for a specific disease type. Customisation of disease specific therapies can be accomplished using either a single strain or mutants or variants thereof or a selection of these strains.

The enteric microbiota is important to the development and proper function of the intestinal immune system. In the absence of an enteric microbiota, the intestinal immune system is underdeveloped, as demonstrated in germ free animal models, and certain functional parameters are diminished, such as macrophage phagocytic ability and immunoglobulin production. The importance of the gut microbiota in stimulating non-damaging immune responses is becoming more evident. The increase in incidence and severity of allergies in the western world has been linked with an increase in hygiene and sanitation, concomitant with a decrease in the number and range of infectious challenges encountered by the host. This lack of immune stimulation may allow the host to react to non-pathogenic, but antigenic, agents resulting in allergy or autoimmunity. Deliberate consumption of a series of non-pathogenic immunomodulatory bacteria would provide the host with the necessary and appropriate educational stimuli for proper development and control of immune function.

### Prebiotics

The introduction of probiotic organisms is accomplished by the ingestion of the micro-organism in a suitable carrier. It would be advantageous to provide a medium that would promote the growth of these probiotic strains in the large bowel. The addition of one or more oligosaccharides, polysaccharides, or other prebiotics enhances the growth of lactic acid bacteria in the gastrointestinal tract. Prebiotics refers to any non-viable food component that is specifically fermented in the colon by indigenous bacteria thought to be of positive value, e.g. bifidobacteria, lactobacilli. For *L. reuteri,* the most promising prebiotic to be used in combination is raffinose and substrates related to raffinose, such as AlphaGOS. The combined administration of a probiotic strain with one or more prebiotic compounds may enhance the growth of the administered probiotic *in vivo* resulting in a more pronounced health benefit, and is termed synbiotic.

### Other active ingredients

It will be appreciated that the probiotic strains may be administered prophylactically or as a method of treatment either on its own or with other probiotic and/or prebiotic materials as described above. In addition, the bacteria may be used as part of a prophylactic or treatment regime using other active materials such as those used for treating inflammation or other disorders especially those with an immunological involvement. Such combinations may be administered in a single formulation or as separate formulations administered at the same or different times and using the same or different routes of administration.

### Pharmaceutical compositions

A pharmaceutical composition is a composition that comprises or consists of a therapeutically effective amount of a pharmaceutically active agent. It preferably includes a pharmaceutically acceptable carrier, diluent or excipients (including combinations thereof). Acceptable carriers or diluents for therapeutic use are well known in the pharmaceutical art, and are described, for example, in Remington's Pharmaceutical Sciences. The choice of pharmaceutical carrier, excipient or diluent can be selected with regard to the intended route of administration and standard pharmaceutical practice. The pharmaceutical compositions may comprise as - or in addition to - the carrier, excipient or diluent any suitable binder(s), lubricant(s), suspending agent(s), coating agent(s), solubilising agent(s), propellants(s).

Examples of pharmaceutically acceptable carriers include, for example, water, salt solutions, alcohol, silicone, waxes, petroleum jelly, vegetable oils, polyethylene glycols, propylene glycol, liposomes, sugars, gelatin, lactose, amylose, magnesium stearate, talc, surfactants, silicic acid, viscous paraffin, perfume oil, fatty acid monoglycerides and diglycerides, petroethral fatty acid esters, hydroxymethyl-cellulose, polyvinylpyrrolidone, and the like.

Where appropriate, the pharmaceutical compositions can be administered by any one or more of: inhalation, in the form of a suppository or pessary, topically in the form of a lotion, solution, cream, ointment or dusting powder, by use of a skin patch, orally in the form of tablets containing excipients such as starch or lactose, or in capsules or ovules either alone or in a mixture with excipients, or in the form of elixirs, solutions or suspensions containing flavouring or colouring agents, or they can be injected parenterally, for example intracavernosally, intravenously, intramuscularly or subcutaneously. For parenteral administration, the compositions may be best used in the form of a sterile aqueous solution which may contain other substances, for example enough salts or monosaccharides to make the solution isotonic with blood. For buccal or sublingual administration the compositions may be administered in the form of tablets or lozenges which can be formulated in a conventional manner.

Intranasal administration can be accomplished using a nasal spray, nasal wash solution or direct application within the nose.

Administration to the lung could be in the form of a dry powder, inhaled using an inhaler device. In some cases the formulation is in the form of an aerosol. The aerosol may be a solution, suspension, spray, mist, vapour, droplets, particles, or a dry powder, for example, using a method dose inhaler including HFA propellant, a metered dose inhaler with non-HFA propellant, a nebulizer, a pressurized can, of a continuous sprayer.

There may be different composition/formulation requirements dependent on the different delivery systems. By way of example, the pharmaceutical composition of the present invention may be formulated to be delivered using a mini-pump or by a mucosal route, for example, as a nasal spray or aerosol for inhalation or ingestible solution, or parenterally in which the composition is formulated by an injectable form, for delivery, by, for example, an intravenous, intramuscular or subcutaneous route. Alternatively, the formulation may be designed to be delivered by both routes.

### References

Abrahamsson TR, Jakobsson HE, Andersson AF, Björkstén B, Engstrand L, & Jenmalm, MC (2012). Low diversity of the gut microbiota in infants with atopic eczema. Journal of allergy and clinical immunology, 129(2), 434-440.

Bisgaard H, Li N, Bonnelykke K, Chawes BLK, Skov T, Paludan-Müller G, ... & Krogfelt KA (2011). Reduced diversity of the intestinal microbiota during infancy is associated with increased risk of allergic disease at school age. Journal of Allergy and Clinical Immunology, 128(3), 646-652.

Blaser MJ, Falkow S. (2009). What are the consequences of the disappearing human microbiota? Nat Rev Microbiol. 7(12):887-894. PMID: 19898491.

Buffington SA, Di Prisco GV, Auchtung TA, Ajami NJ, Petrosino JF, Costa-Mattioli M. (2016). Microbial reconstitution reverses maternal diet-induced social and synaptic deficits in offspring. Cell 165(7): 1762-1775. PMID: 27315483.

Duar RM, Frese SA, Lin XB, Fernando SC, Burkey TE, Tasseva G, Peterson DA, Blom J, Wenzel CQ, Szymanski CM, Walter J (2017). Experimental evaluation of host adaptation of Lactobacillus reuteri to different vertebrate species. Appl Environ Microbiol. 83(12):e00132-17. PMID: 28389535.

Frese SA, Benson AK, Tannock GW, Loach DM, Kim J, Zhang M, Oh PL, Heng NC, Patil PB, Juge N, MacKenzie DA, Pearson BM, Lapidus A, Dalin E, Tice H, Goltsman E, Land M, Hauser L, Ivanova N, Kyrpides NC, Walter J (2011). The evolution of host specialization in the vertebrate gut symbiont Lactobacillus reuteri. PLOS Genetics. 7(2).

Frese SA, MacKenzie DA, Peterson DA, Schmaltz R, Fangman T, Zhou Y, Zhang C, Benson AK, Cody LA, Mulholland F, Juge N, Walter J (2013). Molecular Characterization of Host-Specific Biofilm Formation in a Vertebrate Gut Symbiont. PLOS Genetics.

Hanski I, Von Hertzen L, Fyhrquist N, Koskinen K, Torppa K, Laatikainen T, Karisola P, Auvinen P, Paulin L, Mäkelä MJ, Vartiainen E, Kosunin TU, Alenius H, Haahtela T (2012). Environmental biodiversity, human microbiota, and allergy are interrelated. PNAS. 109(21): 8334-8339.

Hatala T, Laatikainen T, Alenius H, Auvinen P, Fyhrquist N, Hanski I, Von Hertzen L, Jousilahti P, Kosunin TU, Markelova O, Mäkelä MJ, Pantelejev V, Uhanov M, Zilber E, Vartiainen E (2015). Hunt for the origin of allergy - comparing the Finnish and Russian Karelia. Clinical & amp; Experimental Allergy. 45(5).

He B, Hoang TK, Wang T, Ferris M, Taylor CM, Tian X, Luo M, Tran DQ, Zhou J, Tatevian N, Luo F, Molina JG, Blackburn MR, Gomez TH, Roos S, Rhoads JM, Liu Y. (2017). Resetting microbiota by Lactobacillus reuteri inhibits T reg deficiency-induced autoimmunity via adenosine A2A receptors. J Exp Med. 214:107-23. PMID: 27994068.

Jarchum I, Pamer EG. Regulation of innate and adaptive immunity by the commensal microbiota. Curr Opin Immunol 2011;23:353-60.

Kell, Douglas B., et al. "Viability and activity in readily culturable bacteria: a review and discussion of the practical issues." Antonie van Leeuwenhoek73.2 (1998): 169-187

Lamas B, Richard ML, Leducq V, Pham HP, Michel ML, Da Costa G, Bridonneau C, Jegou S, Hoffmann TW, Natividad JM, Brot L, Taleb S, Couturier-Maillard A, Nion-Larmurier I, Merabtene F, Seksik P, Bourrier A, Cosnes J, Ryffel B, Beaugerie L, Launay JM, Langella P, Xavier RJ, Sokol H. (2016). CARD9 impacts colitis by altering gut microbiota metabolism of tryptophan into aryl hydrocarbon receptor ligands. Nat Med. 22(6): 598-605. PMID: 27158904.

Lisciandro JG, Prescott SL, Nadal-Sims MG, Devitt CJ, Richmond PC, Pomat W, ... & van den Biggelaar AH (2012). Neonatal antigen-presenting cells are functionally more quiescent in children born under traditional compared with modern environmental conditions. Journal of Allergy and Clinical Immunology, 130(5), 1167-1174.

Lisciandro, J. G., Prescott, S. L., Nadal-Sims, M. G., Devitt, C. J., Pomat, W., Siba, P. M., & van den Biggelaar, A. H. (2012). Comparison of neonatal T regulatory cell function in Papua New Guinean and Australian newborns. Pediatric Allergy and Immunology, 23(2), 173-180.

Liu Y, Fatheree NY, Dingle BM, Tran DQ, Rhoads, JM (2013). Lactobacillus reuteri DSM 17938 changes the frequency of Foxp3+ regulatory T cells in the intestine and mesenteric lymph node in experimental necrotizing enterocolitis. PloS one, 8(2), e56547.

Liu Y, He B, Hoang TK, Wang T, Taylor CM, Tian X, ... & Luo F (2017). Therapeutic effect of Lactobacillus reuteri DSM 17938 on Treg-deficiency-induced autoimmunity (IPEX syndrome) via the inosine-adenosine 2A receptors.

Marsland JB. (2016). Regulating inflammation with microbial metabolites. Nature Medicine 22:581-583. PMID: 27270775.

Martinez I, Stegen JC, Maldonado-Gómez MX, Eren AM, Siba PM, Greenhill AR, Walter J. (2015). The gut microbiota of rural papua new guineans: composition, diversity patterns, and ecological processes. Cell Rep. 11(4): 527-538. PMID: 25892234.

Mitsuoka T (1992). Intestinal Flora and Aging. Nutrition Reviews 50(12).

Modern Food Biology 2005 7th edition, James Monroe Jay, Martin J. Loessner, David A. Golden, Springer Science, New York.

Oh PL, Benson AK, Peterson DA, Patil PB, Moriyama EN, Roos S, Walter J (2010). Diversification of the gut symbiont Lactobacillus reuteri as a result of host-driven evolution. The ISME Journal. 4: 377-387

Rattanapasert M, Roos S, Hutkins RW, Walter J. Quantitative evaluation of symbiotic strategies to improve persistence and metabolic activity of Lactobacillus reuteri DSM17938 in the human gastrointestinal tract. Journal of Functional Foods. 10:85-94 (2014).

Reuter G (2001). The Lactobacillus and Bifidobacterium microflora of the human intestine: composition and succession. Curr Issues Intest Microbiol. 2(2): 43-53.

Rook G (2013). "The immune system is like an army": An interview with Professor Graham Rook. Gut Microbiota Composition

Ruiz-Núñez B, Pruimboom L, Dijck-Brouwer DA, Muskiet FA. (2013). Lifestyle and nutritional imbalances associated with Western diseases: causes and consequences of chronic systemic low-grade inflammation in an evolutionary context. J Nutr Biochem. 24(7): 1183-1201. PMID: 23657158.

Segata N. (2015). Gut microbiome: Westernization and the disappearance of intestinal diversity. Current Biology. 25(14): R611-R613. PMID: 26196489.

Sinkiewicz G, Cronholm S, Ljunggren L, Dahlén G, Bratthall G (2010). Influence of dietary supplementation with Lactobacillus reuteri on the oral flora of healthy subjects. Swed Dent J. 34(4): 197-206.

Sonnenburg ED, Sonnenburg JL. (2014). Starving our microbial self: the deleterious consequences of a diet deficient in microbiota-accessible carbohydrates. Cell Metab. 2014, 20(5): 779-786. PMID: 25156449.

Spinler JK., Sontakke A, Hollister EB, Venable SF, Oh PL, Balderas MA, ... & Versalovic J (2014). From prediction to function using evolutionary genomics: human-specific ecotypes of Lactobacillus reuteri have diverse probiotic functions. Genome biology and evolution, 6(7), 1772-1789.

van Nimwegen FA, Penders J, Stobberingh EE, Postma DS, Koppelman GH, Kerkhof M, ... & Mommers M (2011). Mode and place of delivery, gastrointestinal microbiota, and their influence on asthma and atopy. Journal of Allergy and Clinical Immunology, 128(5), 948-955.

Walter J, Britton RA, Roos S. (2011). Host-microbial symbiosis in the vertebrate gastrointestinal tract and the Lactobacillus reuteri paradigm. Proc Natl Acad Sci USA 108(1): 4645-4652. PMID: 20615995.

Walter J. Ecological Role of Lactobacilli in the Gastrointestinal Tract: Implications for Fundamental and Biomedical Research. American Society for Microbiology.

Wickens K, Black PN, Stanley TV, Mitchell E, Fitzharris P, Tannock GW, ... & Probiotic Study Group (2008). A differential effect of 2 probiotics in the prevention of eczema and atopy: a double-blind, randomized, placebo-controlled trial. Journal of Allergy and Clinical Immunology, 122(4), 788-794.

Zelante T, Iannitti RG, Cunha C,De Luca A, Giovannini G, Pieraccini G, Zecchi R, D'Angelo C, Massi-Benedetti C, Fallarino A, Puccetti P, Romani L. (2013). Tryptophan catabolites from microbiota engage aryl hydrocarbon receptor and balance mucosal reactivity via interleukin-22. Immunity. 39:372-85. PMID: 23973224.

Zhao X, Gänzle MG (2018). Genetic and phenotypic analysis of carbohydrate metabolism and transport in Lactobacillus reuteri. Int J Food Microbiol. 2;272: 12-21.

## Claims

1. *Lactobacillus reuteri* strain having NCIMB accession number 42835.

2. A strain as claimed in claim 1 wherein the strain is in the form of a biologically pure culture, a bacterial broth, or a freeze dried powder

3. A strain as claimed in any of claims 1 or 2 wherein the strain is a human symbiont.

4. A strain as claimed in any of claims 1 to 3 in the form of viable and/or non-viable cells.

5. A formulation which comprises a strain as claimed in any of claims 1 to 4.

6. A formulation as claimed in claim 5 which further comprises a substrate for the strain.

7. A formulation as claimed in claim 6 wherein the substrate is a carbohydrate.

8. A formulation as claimed in claim 6 or 7 wherein the substrate is an oligosaccharide which may be non-digestible.

9. A formulation as claimed in any of claims 5 to 8 wherein the substrate comprises raffinose.

10. A formulation as claimed in any of claim 5 to 9 further comprising a prebiotic material and/or another probiotic material.

11. A formulation as claimed in any of claims 5 to 10 further comprising an ingestable carrier.

12. A formulation as claimed in claim 11 wherein the ingestable carrier comprises a pharmaceutically acceptable carrier such as a capsule, tablet or powder.

13. A formulation as claimed in claim 11 wherein the ingestable carrier is a food product such as acidified milk, yoghurt, frozen yoghurt, milk powder, milk concentrate, ice cream, cheese spread, dressing or beverage.

14. *Lactobacillus reuteri* strain having NCIMB accession number 42835 for use in enhancing the microbiota of an individual who requires an enhanced microbiota.

15. *Lactobacillus reuteri* strain having NCIMB accession number 42835 for use as claimed in claim 14 wherein the individual is an infant, a young child, a pregnant female or a mother post childbirth.

## Patentansprüche

1. *Lactobacillus-reuteri-Stamm,* der die NCIMB-Zugangsnummer 42835 hat.

2. Stamm nach Anspruch 1, wobei der Stamm in Form einer biologisch reinen Kultur, einer Bakterienbrühe oder eines gefriergetrockneten Pulvers vorliegt.

3. Stamm nach einem der Ansprüche 1 oder 2, wobei der Stamm ein menschlicher Symbiont ist.

4. Stamm nach einem der Ansprüche 1 bis 3 in Form von lebensfähigen und/oder nichtlebensfähigen Zellen.

5. Formulierung, die einen Stamm nach einem der Ansprüche 1 bis 4 umfasst.

6. Formulierung nach Anspruch 5, die weiter ein Substrat für den Stamm umfasst.

7. Formulierung nach Anspruch 6, wobei das Substrat ein Kohlenhydrat ist.

8. Formulierung nach Anspruch 6 oder 7, wobei das Substrat ein Oligosaccharid ist, das unverdaulich sein kann.

9. Formulierung nach einem der Ansprüche 5 bis 8, wobei das Substrat Raffinose umfasst.

10. Formulierung nach einem der Ansprüche 5 bis 9, die weiter einen präbiotischen Stoff und/oder einen weiteren probiotischen Stoff umfasst.

11. Formulierung nach einem der Ansprüche 5 bis 10, die weiter einen einnehmbaren Träger umfasst.

12. Formulierung nach Anspruch 11, wobei der einnehmbare Träger einen pharmazeutisch verträglichen Träger umfasst, wie zum Beispiel eine Kapsel, Tablette oder ein Pulver.

13. Formulierung nach Anspruch 11, wobei der einnehmbare Träger ein Nahrungsmittelprodukt ist, wie zum Beispiel gesäuerte Milch, Joghurt, gefrorener Joghurt, Milchpulver, Milchkonzentrat, Speiseeis, Schmelzkäse, Sauce oder Getränk.

14. *Lactobacillus-reuteri-Stamm,* der die NCIMB-Zugangsnummer 42835 hat, für die Verwendung bei der Verbesserung der Mikrobiota eines Individuums, das eine verbesserte Mikrobiota benötigt.

15. *Lactobacillus-reuteri-Stamm,* der die NCIMB-Zugangsnummer 42835 hat, für die Verwendung nach Anspruch 14, wobei das Individuum ein Säugling, ein Kleinkind, eine Schwangere oder eine Mutter nach der Entbindung ist.

## Revendications

1. Souche de *Lactobacillus reuteri* ayant le numéro d'accès NCIMB 42835.

2. Souche selon la revendication 1, la souche se trouvant sous la forme d'une culture biologiquement pure, d'un bouillon bactérien, ou d'une poudre lyophilisée.

3. Souche selon l'une quelconque des revendications 1 ou 2, la souche étant un symbiote humain.

4. Souche selon l'une quelconque des revendications 1 à 3, sous la forme de cellules viables et/ou non viables.

5. Formulation comprenant une souche selon l'une quelconque des revendications 1 à 4.

6. Formulation selon la revendication 5, comprenant en outre un substrat pour la souche.

7. Formulation selon la revendication 6, dans laquelle le substrat est un glucide.

8. Formulation selon la revendication 6 ou 7, dans laquelle le substrat est un oligosaccharide qui peut être non digestible.

9. Formulation selon l'une quelconque des revendications 5 à 8, dans laquelle le substrat comprend du raffinose.

10. Formulation selon l'une quelconque des revendications 5 à 9, comprenant en outre un matériau prébiotique et/ou un autre matériau probiotique.

11. Formulation selon l'une quelconque des revendications 5 à 10, comprenant en outre un véhicule ingérable.

12. Formulation selon la revendication 11, dans laquelle le véhicule ingérable comprend un véhicule pharmaceutiquement acceptable tel qu'une capsule, un comprimé ou une poudre.

13. Formulation selon la revendication 11, dans laquelle le véhicule ingérable est un produit alimentaire tel qu'un lait acidifié, un yaourt, un yaourt glacé, un lait en poudre, un lait concentré, une crème glacée, un fromage tartinable, une sauce pour salade ou une boisson.

14. Souche de *Lactobacillus reuteri* ayant le numéro d'accès NCIMB 42835, pour une utilisation dans l'amélioration du microbiote d'un individu nécessitant une amélioration de microbiote.

15. Souche de *Lactobacillus reuteri* ayant le numéro d'accès NCIMB 42835 pour une utilisation selon la revendication 14, l'individu étant un nourrisson, un jeune enfant, une femme enceinte ou une mère après l'accouchement.
